(19) Europäisches Patentamt
European Patent Office
Office européen des brevets

(11) **EP 2 399 636 A1**

(12) **EUROPEAN PATENT APPLICATION**

(43) Date of publication:
**28.12.2011 Bulletin 2011/52**

(51) Int Cl.:
*A61M 11/04* (2006.01)     *A61M 15/06* (2006.01)
*A24F 47/00* (2006.01)

(21) Application number: **10251135.9**

(22) Date of filing: **23.06.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Philip Morris Products S.A.
2003 Neuchâtel (CH)**

(72) Inventors:
• **Thorens, Michel
1510 Moudon (CH)**

• **Flick, Jean-Marc
1405 Pomy (CH)**
• **Cochand, Olivier Yves
2056 Dombresson (CH)**
• **Dubief, Flavien
2000 Neutchatel (CH)**

(74) Representative: **Millburn, Julie Elizabeth
Reddie & Grose
16 Theobalds Road
London
WC1X 8PL (GB)**

(54)     **An improved aerosol generator and liquid storage portion for use with the aerosol generator**

(57)     A liquid storage portion (113) comprising an electrical component (504, 505, 804, 904, 905, 906, 907, 1301, 1401) for distinguishing the storage portion (113) from other liquid storage portions is disclosed. The liquid storage portion (113) is configured for use in an aerosol generating system (100) having means for determining (309) an electrical characteristic of the electrical component and means for distinguishing (309) the liquid storage portion (113) from other liquid storage portions based on the determined electrical characteristic of the electrical component.

**Figure 1**

**Description**

The present invention relates to an aerosol generator and to a smoking system. The aerosol generator or smoking system may have a liquid storage portion. More particularly, this invention relates to a liquid storage portion for use with an aerosol generator or a smoking system.

WO-A-2007/078273 discloses a smoking utensil. A liquid is stored in a container which communicates with a heater vaporiser, powered by a supply, via a series of small apertures. The liquid can be fed into the container via a one-way valve. In use, the heater is activated by the mouth of the user to switch on the battery power supply. Suction on a mouthpiece by the user causes air to be drawn through the apertures in the container, over the heater vaporiser, into the mouthpiece and subsequently into the mouth of a user.

One disadvantage of such a proposed smoking utensil is that there is no way to determine that the liquid fed into the container is suitable for use with the smoking utensil. In other words, there is no way of determining whether the liquid fed into the container it is suitable for vaporization by the heater in an appropriate manner.

It is therefore an object of the present invention to overcome this disadvantage and other disadvantages of the prior art.

According to a first aspect of the present invention, there is provided a liquid storage portion comprising an electrical component for distinguishing the storage portion from other liquid storage portions, the liquid storage portion being configured for use in an aerosol generating system having means for determining an electrical characteristic of the electrical component and means for distinguishing the liquid storage portion from other liquid storage portions based on the determined electrical characteristic of the electrical component.

The electrical component may be located on the exterior of the liquid storage portion or within the liquid storage portion.

The liquid storage portion may further comprise one or more further electrical components the components preferably being connected in series or parallel with each other.

The electrical component or components may comprise one or more resistors or one or more radio frequency identification microchips or one or more microchip memories.

The liquid storage portion may further comprise a plurality of electrical contacts for electrical connection to one or more of the electrical component or components.

The electrical contacts may be arranged in a substantially straight line or in a substantially triangular array or in a substantially square or in a substantially rectangular array.

The liquid storage portion may further comprise a heating element. Preferably, the heating element is integral to the liquid storage portion.

In this way, when the liquid storage portion is removed from the aerosol generating system, both the liquid storage portion and the heating element are removed. Then both the liquid storage portion and the heating element which has been removed from the aerosol generating system may be replaced or substituted with another liquid storage portion and heating element.

The material of the heating element may be chosen so that, in use, it heats up more than the electrical contacts or other electrically conductive portions of the liquid storage portion.

The heating element may be electrically connected at a first end to a first end of a first electrically conductive portion. The heating element may be electrically connected at a second end to a first end of a further electrically conductive portion.

The first electrically conductive portion may be electrically connected at a second end to an electrical contact. The second electrically conductive portion may be electrically connected at a second end to an electrical contact.

Usually the means for determining an electrical characteristic of the electrical component is a processor or controller. Further, the means for distinguishing one liquid storage portion from other liquid storage portions based on a determined characteristic of an electrical component is also usually a processor or controller, but a combination of resistors, transistors and other switches may be used.

Electrical components which may be used with embodiments of the invention are one or more resistors, capacitors, inductors, diodes, transistors, memory chips.

The electrical characteristic which may be determined by the processor are for example, the resistance, capacitance, inductance, break down voltage, switching speed or amplification, or in the case of a memory chips, the values stored in the memory.

According to a second aspect of the present invention, there is provided an aerosol generating system comprising the liquid storage portion according to the first aspect of the present invention.

According to a third aspect of the present invention, there is provided an aerosol generating system for receiving a liquid storage portion configured for use with the aerosol generating system, the system comprising: means for determining an electrical characteristic of an electrical component associated with the liquid storage portion; and means for distinguishing the liquid storage portion from other liquid storage portions for use with the aerosol generating system based on the determined electrical characteristic of the electrical component associated with the liquid storage portion.

Preferably, the system further comprises transmission means arranged to transmit signals between the determining means and the electronic component.

The transmission means may be electrical one or more electrical connectors or electrical conductive portions for transmitting electrical signals between the distinguishing means and the electrical component. Alternatively, the transmission means may comprise a radio frequency identification antenna. The antenna may be electrically connected to a radio frequency identification reader or chip on the aerosol generating system. The transmission means may also comprise an antenna associated with a liquid storage portion. The liquid storage portion antenna may be electrically connected to a radio frequency identification chip also associated with the liquid storage portion.

Preferably, the system comprises a housing for at least partially receiving the liquid storage portion. Preferably, the system further comprises means for detecting the presence of the liquid storage portion in the housing.

The means for determining an electrical characteristic of the electrical component may further comprise one or more further electrical components. The one or more further components may be arranged in series with the one or more components associated with the liquid storage portion. The further electrical components on the aerosol generating system may be electrical resistors.

The aerosol generating system may further comprise one or more electrical contacts for electrical connection to the one or more of the further electrical component or components of the aerosol generating system. The aerosol generating system may further comprise one or more electrically conductive potions for electrically connecting the further electrical components or electrical contacts to the determining means and the distinguishing means.

The aerosol generating system may further comprise a memory for storing a look-up table, the look-up table comprising data representing one or more electrical characteristics of one or more electrical components, each characteristic associated with data identifying a liquid storage portion.

The aerosol generating system may further comprise a means for searching the look-up table.

The aerosol generating system may further comprise a means for determining a type of liquid storage portion by searching the look-up table for data identifying the type of liquid storage portion associated with data representing an electrical characteristic of the electronic component which matches data representing the determined characteristic of the electrical component.

The aerosol generating system may further comprise a heating element.

The aerosol generating system may further comprise a means for controlling one or more operational parameters of the heating element. The control means may control one or more of the operational parameters of the heating element in dependence upon the determined electrical characteristic of the electrical component or components associated with the liquid storage portion.

The control means may be arranged to supply electrical energy to the heating element in dependence upon the determined value of the electrical component or components associated with the liquid storage portion.

According to a fourth aspect of the present invention, there is provided a liquid storage portion comprising a 3-dimensional object for distinguishing the storage portion from other liquid storage portions, the liquid storage portion being configured for use in an aerosol generating system having means for determining a characteristic of the 3-dimensional object and means for distinguishing the liquid storage portion from other liquid storage portions based on the determined characteristic of the 3-dimensional object.

According to a fifth aspect of the present invention, there is provided an aerosol generating system for receiving a liquid storage portion configured for use with the aerosol generating system, the system comprising: means for determining a characteristic of a 3-dimensional object associated with the liquid storage portion; and means for distinguishing the liquid storage portion from other liquid storage portions for use with the aerosol generating system based on the determined characteristic of the 3-dimensinoal object associated with the liquid storage portion.

The three dimensional object may comprise one or more bump, protrusions or recesses and the like. The characteristics of the 3-dimensional object may a spatial characteristic of the 3-dimensional object. The characteristic may be the position of the bump, protrusion or recess on the liquid storage portion, the height of a bump or protrusion from the surface of the liquid storage portion, the width of a bump or protrusion from the surface of the liquid storage portion, the depth of a trough or recess in the liquid storage portion. Further, if the 3-dimensional object comprises a plurality of protrusions or recesses, then the relative spacing of the protrusions or recesses may be determined as a characteristic of the 3-dimensional object.

Alternatively, the 3-dimensional object may be engraved or etched into the liquid storage portion. The 3-dimensional object may also be attached to the liquid storage portion by adhesive or other suitable means.

According to a sixth aspect of the present invention, there is provided a method for distinguishing between types of liquid storage portion used with an aerosol generating system, the method comprising the steps of: determining an electrical characteristic of an electrical component associated with the liquid storage portion; distinguishing the liquid storage portion from other liquid storage portions for use with the aerosol generating system based on the determined electrical characteristic of the electrical component associated with the liquid storage portion.

Preferably, the method further comprises the step of comparing the determined electrical characteristic to a look-up table of electrical characteristics of one or more electrical components, each electrical characteristic associated with an identifier of a liquid storage portion. Preferably, the type of liquid storage portion inserted into the aerosol generating system is determined in dependence upon the result of the determination.

The step of determining the electrical characteristic may comprise the steps of: measuring a current flow through a resistor; measuring a voltage across the resistor; and determining the resistance value of the resistor from the determined voltage and current values.

The type of liquid storage portion may be determined as a liquid storage portion identifier which is associated with the electrical characteristic stored in the database which corresponds to the determined characteristic value of the electrical component.

The method may further comprise a step of searching the look-up table or a further look-up table for one or more operational parameters of a heating element which are associated with an electrical characteristic which correspond to the determined characteristic value of the electrical component.

The method may further comprise the step of energizing the heating element according to one or more of the operational parameters which correspond to the determined characteristic value of the electrical component.

The method may further comprise the step of energizing the heating element according to one or more of the operational parameters which correspond to the determined characteristic value of the electrical component.

The method may further comprise the step of energizing the heating element in proportion to the determined characteristic value of the electrical component.

The step of determining an electrical characteristic of an electrical component may be repeated a plurality of times For example, if the liquid storage portion comprises 4 resistors, then the step of determining the electrical characteristic is repeated 4 times.

Preferably, a heating element is energized according to one or more of the operational parameters, the value of each operation parameter applied to the heating element being proportional to the determined electrical characteristic of one or the components.

Preferably, one the characteristic value of one electrical component determines the value of one operational parameter applied to the heating element.

Preferably, the step of determining the electrical characteristic comprises the steps of: interrogating a chip; receiving a signal from the chip, the signal comprising electrical characteristic data of the chip; and determining the type of liquid storage portion from the received electrical characteristic data.

The chip may be a radio frequency identification chip which is interrogated by energizing it with a radio-frequency signal. The signal received from the chip may be a radio frequency signal.

Alternatively, the chip may be an electrically erasable programmable read only memory which is interrogated by wired electrical connection to a processor or controller. The signal may be received from the chip by a wired electrical connection.

The data may comprise a liquid storage portion identifier and optionally one or more operational parameters of the heating element which the system may use to energize the heating element.

Preferably, the electrical contacts may be made out of an electrically conductive material with low resistivity, such as copper, silver or gold.

The electrically conductive means may be an electrically conductive material with low resistivity, such as copper, silver or gold. This minimises energy loss in this portion of the smoking system.

According to a seventh aspect of the present invention, there is provided a method for distinguishing between types of liquid storage portion used with an aerosol generating system, the method comprising the steps of determining a characteristic of a 3-dimensional object associated with the liquid storage portion distinguishing the liquid storage portion from other liquid storage portions for use with the aerosol generating system based on the determined characteristic of the e3-dimensional object associated with the liquid storage portion (113). When the liquid storage portion is new, it is filled with a suitable liquid for producing an aerosol. In use, the aerosol generating system uses liquid contained or held within the liquid storage portion to produce the aerosol. The aerosol generating system uses the liquid contained in the liquid storage portion until the liquid storage portion is empty or substantially empty. Therefore, the liquid storage portion may contain some liquid or may be empty so that no liquid is contained within the liquid storage portion.

In use, when the heater is activated, liquid provided by the liquid storage portion is vaporized by the heater to form a supersaturated vapour. The supersaturated vapour is mixed with and carried in the air flow from the at least one air inlet. During the flow, the vapour condenses to form an aerosol in the chamber, and the aerosol is carried towards the air outlet into the mouth of a user. In this specification, the upstream and downstream relative positions are described in relation to the direction of air flow as it is drawn from the air inlet to the air outlet.

The liquid may be provided by at least one portion of a capillary wick.

Alternatively or in addition to a heating element, the aerosol generating system may comprise an atomiser to create the aerosol. The atomiser may include one or more electromechanical elements such as piezoelectric elements. Additionally or alternatively, the atomiser may also include elements that use electrostatic, electromagnetic or pneumatic effects.

The smoking system according to embodiments of the invention provides a number of advantages.

The liquid has physical properties, for example a boiling point suitable for use in the smoking system: if the boiling point is too high, the at least one heater will not be able to vaporize liquid in the capillary wick, but, if the boiling point is too low, the liquid may vaporize even without the at least one heater being activated. The liquid preferably comprises a tobacco-containing material comprising volatile tobacco flavour compounds which are released from the liquid upon heating. Alternatively, or in addition, the liquid may comprise a non-tobacco material. The liquid may include water, solvents, ethanol, plant extracts and natural or artificial flavours. Preferably, the liquid further comprises an aerosol former. Examples of suitable aerosol formers are glycerine and propylene glycol.

Further, as known to those skilled in the art, an aerosol is a suspension of solid particles or liquid droplets in a gas, such as air. The aerosol may be a suspension of solid particles and liquid droplets in a gas, such as air.

In a preferred embodiment of the invention, the smoking system comprises a liquid storage portion. Preferably, the capillary wick is arranged to be in contact with liquid in the liquid storage portion. In that case, in use, liquid is transferred from the liquid storage portion towards the heater by capillary action in the capillary wick. In one embodiment, the capillary wick has a first end and a second end, the first end extending into the liquid storage portion for contact with liquid therein and the at least one heater being arranged to heat liquid in the second end. When the heater is activated, the liquid at the second end of the capillary wick is vaporized by the heater to form the supersaturated vapour.

Using a capillary wick extending between the liquid and the heater, allows the structure of the system to be relatively simple. The liquid has physical properties, including viscosity, which allow the liquid to be transported through the capillary wick by capillary action caused by surface tension of the liquid.

The liquid storage portion is preferably a container. Preferably, the liquid storage portion does not include any porous materials, so that there is only a single capillary mechanism, the capillary wick, in the smoking system. This keeps the structure of the smoking system simple and the entire system low-maintenance. Preferably, the container is opaque, thereby limiting degradation of the liquid by light. The liquid storage portion may not be refillable. Thus, when the liquid in the liquid storage portion has been used up, the liquid storage portion is replaced. Alternatively, the liquid storage portion may be refillable. In that case, the liquid storage portion may be replaced after a certain number of refills of the liquid storage portion. Preferably, the liquid storage portion is arranged to hold liquid for a pre-determined number of puffs.

The capillary wick may have a fibrous or spongy structure. For example, the capillary wick may comprise a plurality of fibres or threads. The fibres or threads may be generally aligned in the longitudinal direction of the smoking system. Alternatively, the capillary wick may comprise sponge-like material formed into a rod shape. The rod shape may extend along the longitudinal direction of the smoking system. The structure of the wick forms a plurality of small bores or tubes, through which the liquid can be transported to the heater, by capillary action. The capillary wick may comprise any suitable material or combination of materials. Examples of suitable materials are ceramic- or graphite-based materials in the form of fibres or sintered powders. The capillary wick may have any suitable capillarity and porosity so as to be used with liquids of different physical properties such as density, viscosity, surface tension and vapour pressure. The capillary properties of the wick, combined with the properties of the liquid, ensure that the wick is always wet in the heating area.

Preferably, the smoking system comprises an elongate housing and the longitudinal axis of the capillary wick and the longitudinal axis of the housing are substantially parallel.

The at least one heater may comprise a single heating element. Alternatively, the at least one heater may comprise more than one heating element, for example two, three, four, five, six or more heating elements. The heating element or heating elements may be arranged appropriately so as to most effectively vaporize liquid in the capillary wick.

The at least one heater preferably comprises an electrical heating element. The at least one heater preferably comprises an electrically resistive material. Suitable electrically resistive materials include but are not limited to: semiconductors such as doped ceramics, electrically conductive ceramics such as molybdenum disilicide, carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel-, Constantan-, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal® and iron-manganese-aluminium based alloys. Timetal® is a registered trade mark of Titanium Metals Corporation, 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required.

The at least one heater may take any suitable form. For example, the at least one heater may take the form of a coiled heating element. For example, the heater may take the form of a wire wound coiled heating element. The coil of wire at least partially surrounds the wick. Other alternatives include a heating wire or filament, for example a Ni-Cr, platinum, tungsten or alloy wire.

In that embodiment, preferably the wire is a metal wire. Even more preferably, the wire is a metal alloy wire. The coil may extend fully or partially along the length of the capillary wick. The coil may extend fully or partially around the circumference of the capillary wick. In a preferred embodiment, the coil is in contact with the capillary wick.

Alternatively, the at least one heater may take the form of a casing or substrate having different electro-conductive portions, or an electrically resistive metallic tube. Alternatively, the at least one heater may be a disk or end heater or a combination of a disk heater with heating needles or rods. Alternatively, the at least one heater may take the form of a metallic etched foil insulated between two layers of an inert material. In that case, the inert material may comprise Kapton®, all-polyimide or mica foil. Kapton® is a registered trade mark of E.I. du Pont de Nemours and Company, 1007 Market Street, Wilmington, Delaware 19898, United States of America.

Alternatively, the at least one heater may take the form of a sheet of material, which may be rolled around at least a portion of the capillary wick. Alternatively, the at least one heater may take the form of an etched foil folded around at least a portion of the capillary wick. The etched foil may comprise a metal sheet cut by a laser or by electro-chemical process. The sheet may be made from any suitable material, for example an iron-aluminium based alloy, an iron-manganese-aluminium base alloy or Timetal®. The sheet may be rectangular in shape, or may have a patterned shape which may form a coil-like structure when rolled around the capillary wick.

The at least one heater may heat the liquid in the capillary wick by means of conduction or convection or conduction and convection. Alternatively, heat from the heater may be conducted to the liquid by means of a heat conductive element. Alternatively, the at least one heater may transfer heat to the incoming ambient air that is drawn through the smoking system during use, which in turn heats the liquid by convection. The ambient air may be heated before passing through the system. Alternatively, the ambient air may be first drawn through the wick and then heated.

In one embodiment, the aerosol generating system is a smoking system. Preferably, the aerosol generating system or the smoking system is an electrically heated aerosol generating system or electrically heated smoking system. In that embodiment, the smoking system may further comprise an electric power supply. Preferably, the electric power supply comprises a cell contained in a housing. The electric power supply may be a Lithium-ion battery or one of its variants, for example a Lithium-ion polymer battery. Alternatively, the power supply may be a Nickel-metal hydride battery, a Nickel cadmium battery, a Lithium-maganese battery, a Lithium-cobalt battery or a fuel cell. In that case, preferably, the electrically heated smoking system is usable by a smoker until the energy in the power cell is used up. Alternatively, the electric power supply may comprise circuitry chargeable by an external charging portion. In that case, preferably the circuitry, when charged, provides power for a pre-determined number of puffs, after which the circuitry must be reconnected to the external charging portion. An example of suitable circuitry is one or more capacitors or rechargeable batteries.

If the smoking system is an electrically heated smoking system, the smoking system may further comprise electric circuitry. In one embodiment, the electric circuitry comprises a sensor to detect air flow indicative of a user taking a puff. The sensor may be an electro-mechanical device. Alternatively, the sensor may be any of: a mechanical device, an optical device, an opto-mechanical device, a micro electro mechanical systems (MEMS) based sensor and an acoustic sensor. In that case, preferably, the electric circuitry is arranged to provide an electric current pulse to the at least one heater when the sensor senses a user taking a puff. Preferably, the time-period of the electric current pulse is pre-set, depending on the amount of liquid desired to be vaporized. The electric circuitry is preferably programmable for this purpose.

Alternatively, the electric circuitry may comprise a manually operable switch for a user to initiate a puff. The time-period of the electric current pulse is preferably pre-set depending on the amount of liquid desired to be vaporized. The electric circuitry is preferably programmable for this purpose.

In one embodiment, the at least one air inlet comprises two air inlets. Alternatively, there may be three, four, five or more air inlets. Preferably, if there is more than one air inlet, the air inlets are spaced around the housing. In a preferred embodiment, the electric circuitry comprises a sensor to detect air flow indicative of a user taking a puff, and the at least one air inlet upstream of the sensor.

Preferably, the smoking system further comprises a puff indicator for indicating when the at least one heater is activated. In the embodiment in which the electric circuitry comprises a sensor to detect air flow indicative of a user taking a puff, the indicator may be activated when the sensor senses air flow indicative of the user taking a puff. In the embodiment in which the electric circuitry comprises a manually operable switch, the indicator may be activated by the switch.

The electrically heated smoking system may further comprise an atomiser including the at least one heater. In addition to a heating element, the atomiser may include one or more electromechanical elements such as piezoelectric elements. Additionally or alternatively, the atomiser may also include elements that use electrostatic, electromagnetic or pneumatic effects.

Preferably, the smoking system comprises a housing. The housing may comprise a shell and a mouthpiece. In that case, all the components may be contained in either the shell or the mouthpiece. In the case of an electrically heated smoking system, preferably, the electric power supply and the electric circuitry are contained in the shell. Preferably, the liquid storage portion, the capillary wick, the at least one heater and the air outlet are contained in the mouthpiece. The at least one air inlet may be provided in either the shell or the mouthpiece. Preferably, the mouthpiece is replaceable. Having a shell and a separate mouthpiece provides a number of advantages. Firstly, if the replaceable mouthpiece contains the at least one heater, the liquid storage portion and the wick, all elements which are potentially in contact with the liquid are changed when the mouthpiece is replaced. There will be no cross-contamination in the shell between different mouthpieces, for example ones using different liquids. Also, if the mouthpiece is replaced at suitable intervals, clogging of the heater with liquid is avoided. Preferably, the shell and mouthpiece are arranged to releasably lock together when engaged.

The housing may comprise any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. Preferably, the material is light and non-brittle.

Preferably, the smoking system is portable. The smoking system may have a size comparable to a conventional cigar or cigarette. The smoking system may have a total length between approximately 30 mm and 130 mm. The smoking system may have an external diameter between approximately 5 mm and approximately 13 mm.

Features described in relation to one aspect of the invention may also be applicable to another aspect of the invention.

The invention will be further described, by way of example only, with reference to the accompanying drawings, in which:

Figure 1 shows an aerosol generator according to an embodiment of the invention having a liquid storage portion;
Figure 2 shows an enlarged portion of the aerosol generator of Figure 1;
Figure 3 shows a detailed sectional view of liquid storage portion embodying the invention;
Figure 4 shows a detailed sectional view of an the aerosol generator of Figure 2 with a liquid storage portion inserted into the aerosol generator;
Figure 5 shows a sectional view of a liquid storage portion embodying the invention;;
Figure 6 is a sectional view of another liquid storage portion embodying the invention;
Figure 7 is a schematic circuit diagram showing the wiring of components of one aerosol generator embodying the invention
Figure 8 is a flow chart of the main steps performed by an embodiment of the invention when detecting the presence of liquid storage portion;
Figure 9 is a flow chart of the mains steps performed by an embodiment of the invention when determining the type of liquid storage portion inserted into the aerosol generating system;
Figure 10 is a schematic circuit diagram showing the wiring of components of another aerosol generator embodying the invention;
Figure 11 is a schematic circuit diagram showing the wiring of components of a further aerosol generator embodying the invention;
Figure 12 is a sectional view of a further liquid storage portion embodying the invention;
Figure 13 is a schematic diagram of selected components of an aerosol generating system embodying the invention in which data is stored on a RFID chip; and
Figure 14 is a schematic diagram of selected components of an aerosol generating system embodying the invention in which data is stored on memory.

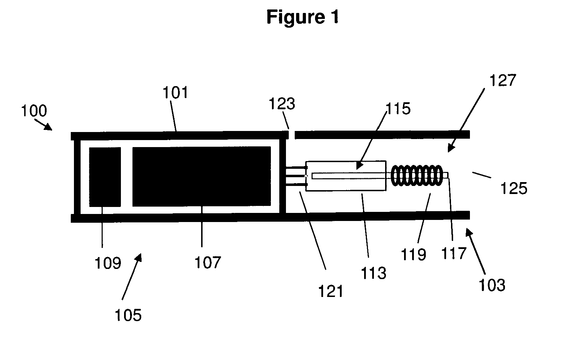

In the drawings, like features are given like reference numbers. Note that Figures 1 to 14 are schematic in nature. In particular, the components shown are not to scale either individually or relative to one another.

Referring now to Figure 1, this shows one example of an aerosol generating system having a liquid storage portion 113. The aerosol generating system 100 may be a smoking system having a liquid storage portion 113. The aerosol generating system 100 of Figure 1 is an electrically heated aerosol generating system 100 and comprises a housing 101 having a mouthpiece end 103 and a body end 105. In the body end 105, there is provided an electric power supply in the form of a battery 107 and electric circuitry in the form of circuitry 109 and a puff detection system, not shown. In the mouthpiece end 103, there is provided a liquid storage portion or cartridge 113 containing liquid 115, a capillary wick 117 and a heating element in the form of a heating coil 119. A first end of the capillary wick 117 extends into the cartridge 113 and a second end of the capillary wick 117 is at least partially surrounded by the heating coil 119. The heating element 119 may be in close proximity to the capillary wick 117. The heating coil 119 is connected to the electric circuitry via connections 121. The housing 101 also includes an air inlet 123, an air outlet 125 at the mouthpiece end and a chamber in the form of aerosol forming chamber 127.

In use, operation is as follows. Liquid 115 is transferred by capillary action from the cartridge 113 from one end of the wick 117 which extends from the cartridge to another end of the wick 117 which is surrounded by the heating coil 119. When a user draws on the device at the air outlet 125, ambient air is drawn through air inlet 123. In the arrangement shown in Figure 1, the puff detection system senses a puff and activates the heating coil 119. The battery 107 supplies electrical energy to the heating coil 119 to heat the end of the wick 117 surrounded by the heating coil. The liquid in that end of the wick 117 is vaporized by the heating coil 119 to create a supersaturated vapour. At the same time, the vaporized liquid is replaced by further liquid which is conveyed along the wick 117 by capillary action. This process is sometimes referred to as pumping action. The supersaturated vapour created is mixed with and carried in the air flow from the air inlet 123. In the aerosol forming chamber 127, the vapour condenses to form an inhalable aerosol, which is carried towards the outlet 125 and into the mouth of the user.

In the embodiment shown in Figure 1, the circuitry 109 and the puff detection system are preferably programmable. The circuitry 109 and puff detection system can be used to manage the device operation. This, in conjunction with the physical design of the electrically heated smoking system, can assist with control of the particle size in the aerosol.

The capillary wick can be made from a variety of porous or capillary materials and preferably has a known, pre-defined capillarity. Examples include ceramic- or graphite-based materials in the form of fibres or sintered powders. Wicks of different porosities can be used to accommodate different liquid physical properties such as density, viscosity, surface tension and vapour pressure. The capillary properties of the wick should be chosen so that the required amount of liquid can be delivered to portion of the wick adjacent to, or in contact with the heating coil.

Figure 1 shows an example of an aerosol generating system which may be used with the present invention. Many other examples are usable with the invention, however. For example, the aerosol generating system need not be electrically operated. Furthermore, a puff detection system need not be provided. Instead, the system may be manually operated, for example, the user may operate a switch to supply electrical energy to the coil when a puff is taken. The housing may also comprise a separable shell and mouthpiece. The overall shape and size of the housing may also be altered from that shown in Figure 1.

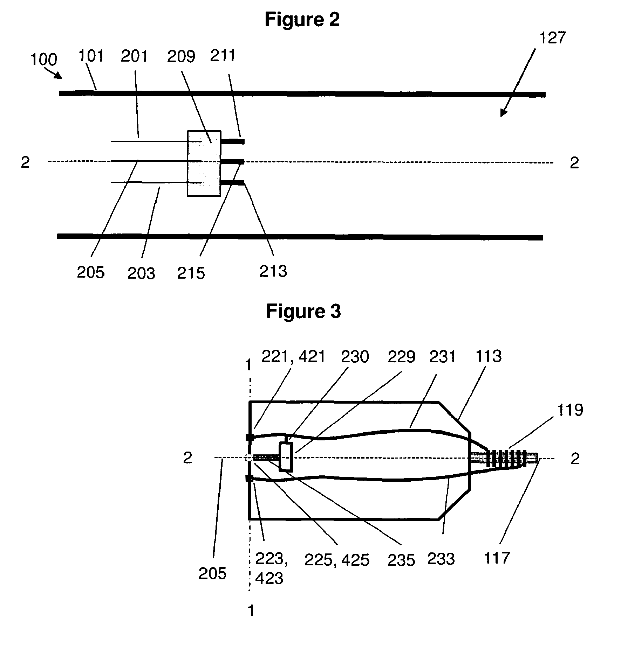

Referring now to Figure 2, this shows an enlarged portion of the aerosol generating system 100 of Figure 1, with the liquid storage portion 113 removed. Three electrical connectors 211, 213, 215 are provided. The electrical connectors are attached, either directly or indirectly to the housing 101. As explained in further detail below, the electrical connectors provide electrical signals to the liquid storage portion 113. The electrical connectors shown Figure 2 are mounted in a holder 209. This maintains the relative positions of the connectors, although the holder is optional.

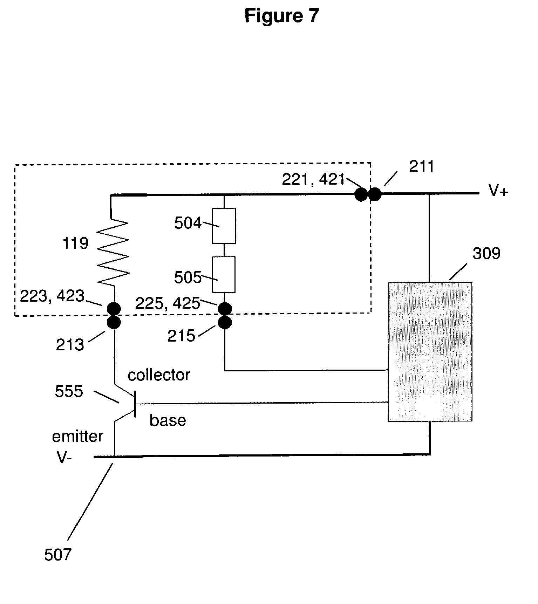

A first electrically conductive element 201 is electrically connected at a first end to a first end of the first electrical connector 211. A second electrically conductive element 203 is electrically connected at a first end to a first end of the second electrical connector 213. A third electrically conductive element 205 is electrically connected at a first end to a first end of the third electrical connector 215. As described in further detail with reference to Figure 7 below, the electrically conductive elements 201, 203, 205 are electrically connected to the electronic circuitry 109, not shown in Figure 2. The electrically conductive elements 201, 203, 205 and electrical connectors 211, 213, 215 allow electrical signals to pass between the circuitry 109 and liquid storage portion 113. Further, the electrically conductive elements 201, 203, 205 are electrically connected to a controller or processor 309 which may be mounted in circuitry 109, not shown in Figure 2, as well as to a power source 107.

The electrical connectors 211, 213, 215 and electrically conductive elements 201, 203, 205 may be directly mounted on or within the housing 101, without the need for a holder 209.

Further, the electrical connectors 211, 213, 215 may be directly electrically connected to the controller or processor 309 and power source 107, without the need for electrically conductive elements 201, 203, 205.

As explained in further detail below, the processor 309 determines whether a liquid storage portion 113 is present in the system 100. The processor 309 may also determine what type of liquid storage portion is present in the system. The controller or processor 309 also controls when electrical power is provided to the coil 119, and this will be explained in further detail below with reference to Figure 7.

Referring now to Figure 3, this shows a detailed section taken through a liquid storage portion 113 according to an embodiment of the invention. The liquid storage portion 113 comprises first, second and third electrical contacts 221 or 421, 223 or 423, 225 or 425 at a first end of the liquid storage portion. The first, second and third electrical contacts 221 or 421, 223 or 423, 225 or 425 are physically mounted on the liquid storage portion 113. The first electrical contact 221 or 421 is electrically connected to a first end of a first electrically conductive portion 231. The second electrical contact 223 or 423 is electrically connected to a first end of a second electrically conductive portion 233. A second end of the first electrically conductive portion 231 is electrically connected to a first end of the heating element 119. A second end of the second electrically conductive portion 233 is electrically connected to a second end of the heating element 119. The electrical contacts 221 or 421, 223 or 423 and electrically conductive portions 231, 233 allow electrical energy to be supplied to the heating element 119 from a battery or other power source, not shown in Figure 2. The battery or power source 107 is usually mounted on or within the aerosol generating system 100.

A liquid storage portion identification subsystem 229 is mounted on or within the liquid storage portion 113. The subsystem may be mounted on the exterior of the liquid storage portion 113 to keep the subsystem and liquid contained within the liquid storage portion 113 separate. The subsystem 229 allows the aerosol generating system 100 to detect when a liquid storage portion 113 is present in the housing 101 of the aerosol generating system 100. Further, the subsystem 229 also allows the aerosol generating system 100 to distinguish between different types of liquid storage portions 113 inserted into the aerosol generating system 100. The subsystem 229 is described in further detail below.

The subsystem 229 is electrically connected to a first end of a third electrically conductive portion 235. The second end of the third electrically conductive portion 235 is electrically connected to a third electrical contact 225, 425 on the liquid storage portion 113.

The subsystem 229 is also electrically connected to the electrically conductive portion 231 with a fourth electrically conductive portion 230, and the purpose of this connection is described below with reference to Figure 7 below.

The electrically conductive portions 201, 203, 205, 230, 231, 233, 235, may be electrically conductive wire, such as a metallic wire, for example copper wire. Further, the electrically conductive portion may be a conductor of low resistivity, such as copper, or silver, or gold.

In the embodiment shown in Figure 3, there are 3 electrical contacts 221, 223, 225, mounted on the liquid storage portion. Further, as previously described, 3 electrical contacts 211, 213, 215, are also provided on the aerosol generating system 100. The electrical contacts 211, 213, 215, 221, 223, 225 may be made out of an electrical conductor. The conductor may have a low resistivity. The conductor may be copper, silver or gold.

When a liquid storage portion is inserted into the aerosol generating system, electrical contacts 221, 223 on the liquid storage portion and two electrical contacts 211, 213 on the aerosol generating system 100 allow electrical energy to be transmitted to the heating element 119. Further, an electrical contact 225 on the liquid storage portion 113 and an electrical contact 215 on the aerosol generating system 100 allow electrical signals to be exchanged between the liquid storage portion 113 and the aerosol generating system 100. This allows a controller 309, not shown in Figure 2, to detect whether a liquid storage portion 113 has been inserted into the aerosol generating system 100. It also allows the controller 309 to distinguish between different types of liquid storage portions 113. This is described in further detail below.

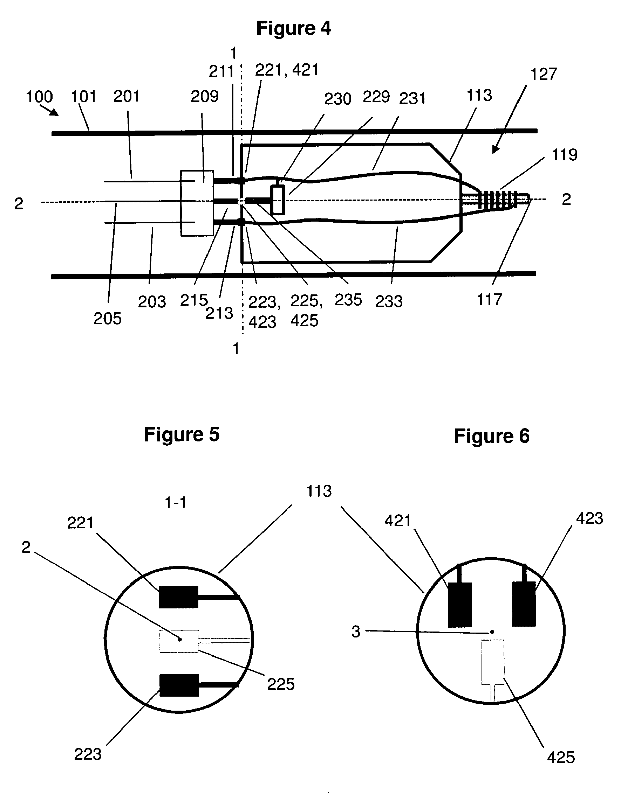

Figure 4 shows the liquid storage portion 113 of Figure 3 inserted into the aerosol generating system 100 of Figure 2. The electrical contacts 221 or 421, 223 or 423, 225 or 425, on the liquid storage portion 113 are arranged so that they contact corresponding electrical contacts 211, 213, 215, on the aerosol generating system 100 when the liquid storage portion 113 is inserted into the aerosol generating system 100. Furthermore, the cartridge or liquid storage portion 113 shown in Figure 4 is contained within the housing 101 of the aerosol generating system 100, however other configurations in which the liquid storage portion 113 is not contained within the housing 101 are possible. In the embodiment shown in Figure 4, the liquid storage portion 113 is replaceable.

As previously described, the electrical contacts 211, 213, 215 are attached to the aerosol generating system 100 to prevent relative movement of the housing 101 and the electrical contacts 211, 213, 215. Further, the electrical contacts 221, 223, 225 are attached to the to the liquid storage portion 113. When the liquid storage portion 113 is removed from the aerosol generating system 100, for example by pulling the liquid storage portion 113 and housing 101 in substantially opposite directions such that the liquid storage portion 113 moves away from the aerosol generating system 100, the electrical connection between contacts 221 or 421, 223 or 423, 225 or 425 on the liquid storage portion 113 and the electrical contacts 211, 213, 215 on the aerosol generating system is broken. Conversely, when the liquid storage portion 113 is inserted in to the aerosol generating system 100, for example by pushing the liquid storage portion 113 and housing 101 in substantially opposite directions such that the liquid storage portion 113 moves towards the aerosol generating system 100, the electrical connection between contacts 221 or 421, 223 or 423, 225 or 425 on the liquid storage portion 113 and the electrical contacts 211, 213, 215 on the aerosol generating system 100 is made. Therefore, the electrical connection between the liquid storage portion 113 and the aerosol generating is only made when a liquid storage portion 113 is inserted into the aerosol generating system 100.

The liquid storage portion 113, wick 117, heating element 119, contacts 221, 223, 225, 421, 423, 425 and electrically conductive portions 230, 231, 233, 235, and subsystem 229 may form a single unit which is disposable. The unit may also be replaceable.

Figures 5 and 6 show two alternative arrangements of the electrical contacts 221, 223, 225, 421, 423, 425 on the liquid storage portion 113.

In one embodiment shown in Figure 5, the electrical contacts 221, 223, 225 on the liquid storage portion are arranged in a row or substantially straight line. Figure 5 is a section through line 1-1 in Figure 4 of the drawings.

Figure 5 shows that the two electrical contacts 221, 223 which are furthest away from the longitudinal axis of symmetry of the liquid storage portion, shown by a dot marked 2 in Figure 5, are the electrical contacts which provide electrical energy to the heating element 119. The electrical contact 225 which is closest to the longitudinal axis of symmetry of the aerosol generating system is the electrical contact which provides the electrical signals to the liquid storage portion recognition subsystem 229.

The electrical contacts mounted on the aerosol generating system are arranged in a similar arrangement to the electrical contacts mounted on the liquid storage portion 113, shown in Figure 5. Therefore, the corresponding electrical contacts 211, 213, 215 on the aerosol generating system 100 are also arranged in a row or substantially straight line. In this embodiment, each electrical contact 211, 213, 215 is equidistant from its neighbouring contact.

Further, the two electrical contacts 211, 213 on the aerosol generating system 100 which are furthest away from the longitudinal axis of symmetry of the aerosol generating system 100, shown by a dotted line marked 2-2 in Figure 4, are the electrical contacts which provide electrical energy to the heating element 119. The electrical contact 215 which is closest to the longitudinal axis of symmetry of the aerosol generating system 100 is the electrical contact which provides the electrical signals to the liquid storage portion recognition subsystem 209.

In a further embodiment, the position of the electrical contacts 421, 423, 425 on the liquid storage portion 113 are in the form of a substantially triangular lattice. The upper two contacts 421, 423 shown in Figure 6 are electrical contacts which transmit electrical energy to the heating element 119, while the lower contact 425 transmits the electrical signals to the liquid storage portion recognition subsystem 229. In this embodiment, the spacing of the centre of each contact 421, 423, 425, from the central axis of symmetry, shown by the dot marked 3 in Figure 6, may be equal to each other or equidistant.

In this embodiment, the electrical contacts mounted on the aerosol generating system are arranged in a similar arrangement to the electrical contacts mounted on the liquid storage portion 113 shown in Figure 6. The position of the electrical contacts 211, 213, 215 mounted on the aerosol generating system are also in the form of a substantially triangular lattice. The contacts 211, 213 shown in Figure 4 which are furthest away from the longitudinal axis of symmetry shown by the dotted line marked 2-2 in Figure 4 are electrical contacts which transmit electrical energy to the heating element 119, while the contact 215 closest to the longitudinal axis of symmetry shown by the dotted line marked 2-2 transmits the electrical signals to the liquid storage portion recognition subsystem 229. In this embodiment, the spacing of the centre of each contact 211, 213, 215 from the central axis of symmetry, shown by the dashed line shown 2-2 in Figure 4 may be equal to each other or equidistant.

Figure 7 shows an electrical circuit diagram of an embodiment of the invention. In Figure 7, the electrical contacts 211, 213, 215, 221, 223, 225, 421, 423, 425, also shown in Figures 2 to 6 are represented by solid dark circles.

Further, the components enclosed by the dashed line enclosure shown in Figure 7 are components which are located on or in the liquid storage portion 113. The components located outside the dashed line enclosure are components which are located on or in the aerosol generating system 100. Therefore, Figure 7 shows the situation where a liquid storage portion is inserted 113 aerosol generating system 100.

Therefore, the components within the enclosure may be replaced each time a liquid storage portion 113 is removed from the aerosol generating system 100 and replaced with a new liquid storage portion 113. The components outside the dashed line, as well as the aerosol generating system 100 itself may be reused a number of times with replacement liquid storage portions 113.

The electrical circuitry of Figure 7 may be divided into 2 separate parts, each of which has a separate function which may be used on its own or in conjunction with one or more of the other parts or other embodiments of the invention.

One part of the electrical circuitry is a liquid storage portion identification part, and this part comprises two electrical resistors, 504 and 505. Although the resistors 504, 505 shown in Figure 7 are connected in series, other configurations are possible, such as parallel connection. Alternatively, a single electrical resistor may be used.

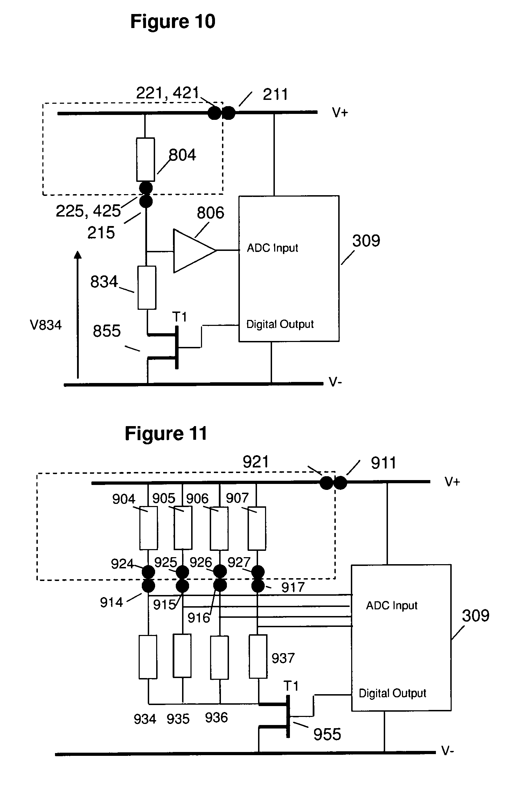

In addition, embodiments of the invention may also use one or more other electrical components such as one or more capacitors, one or more inductors, one or more diodes or one or more transistors and the like, alternatively or in addition to the resistors 504, 505. This is described in further detail with reference to Figures 10 and 11 below.

In Figure 7, a first terminal of a first resistor 504 is electrically connected to a first terminal of the power source V+ via electrical contacts 211, 221, 421. A second terminal of a first resistor 504 is electrically connected to a first terminal of a second resistor 505. The second resistor 505 is electrically connected in series with the first resistor 504. A second terminal of the second resistor 505 is connected to a controller or processor 309 via electrical contacts 215, 225, 425.

When the liquid storage portion is inserted in to the aerosol generating system, the electrical contacts 221 or 421, 223 or 423, 225 or 425 on the liquid storage portion 113 come in to physical contact with the electrical contacts 211, 213, 215 on the aerosol generating system 100. The controller or processor 309 then detects that an electrical circuit has been made because an electrical current can flow through resistors 504, 505. This is because an electrical circuit is made between one terminal of the battery via the resistors 504, 505, and through the controller to the other terminal of the battery which is also connected to the controller. The controller 309 is also electrically connected to a negative terminal, V- of a battery or other power source via an electrically conductive element to complete the electrical circuit.

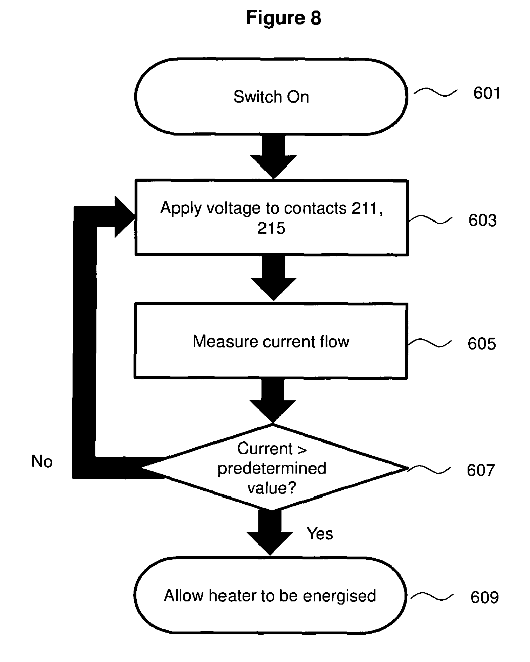

The detailed steps performed by an embodiment of the invention to detect the presence of a liquid storage portion 113 are shown in Figure 8.

At step 601, the aerosol generating device is switched on. At step 603, the controller applies a voltage or potential difference across contacts 211 and 215. Because the resistors 504, 505 are electrically connected to the contacts 225, 425 and contacts 221, 421, the same voltage is also applied across the resistors 504 and 505 assuming that the voltage drop across the contacts is negligible.

At step 605, the controller measures the current flow, if any, flowing through the resistors. At step 607, the controller determines whether the current is greater than a predetermined threshold current. If the measured current is greater than a predetermined value, then the controller determines that a liquid storage portion is present, and allows the heater to be energised at step 609.

By detecting this current, the processor determines that a liquid storage portion 113 has been inserted into the aerosol generating system 100. This is advantageous since the aerosol generating system 100 may be de-activated or switched of unless a liquid storage portion is inserted into the aerosol generating system 100. This saves power and also increases the safety of the system.

Preferably, the aerosol generating system 100 further comprises a puff detector, although this is not shown in Figure 7. The puff detector may be electrically connected to the controller such that the puff detector feeds an electrical signal into the processor or controller 309 which is indicative of when a user takes a puff. The controller 309 may be arranged to only energise the heater if a puff is detected and if the current flow through the resistors 504, 505 is greater than a predetermined threshold current.

Alternatively, the system may be manually operated. For example, the user may operate a switch to supply electrical energy to the heating element 119 when a puff is taken. In this case, the controller 309 may be arranged to only energise the heater if the user closes a switch and if the current flow through the resistors 504, 505 is greater than a predetermined threshold current.

In one embodiment, the controller 309 is able to distinguish between different liquid storage portions 113 inserted in to the aerosol generating system 100.

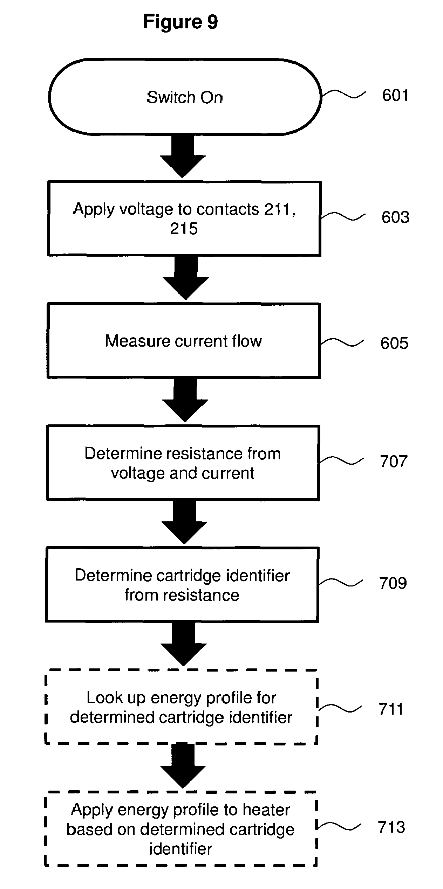

As will be known to those skilled in the art, the voltage drop V across a resistor of resistance R is proportional to the product of the resistance R and the current $I$ flowing through the resistor. By measuring the magnitude or size of the electrical current passing through the resistors 504, 505 for a given potential difference applied across these resistors, the controller 309 is able to determine the value of the sum of the resistances 504, 505 using the relationship $R = V/I$. The detailed steps performed by an embodiment of the invention in determining the type of liquid storage portion 113 are shown in Figure 9. Steps 601, 603, and 605 are the same as those described with reference to Figure 8, and so will not be described again.

At step 707, the controller 309 uses the value of the potential difference applied across the resistors 504 and 505 as well as the measured current flowing through the resistors 504 and 505 to determine the value of the sum of the resistances 504, 505, as previously described.

Having determined the value of the sum of the resistors associated with the liquid storage portion, the controller 309 determines the liquid storage portion type from the determined resistance value by searching a look-up table using the determined resistance value.

The look-up table may comprise one or more different resistance values, each resistance value associated with an identifier of a liquid storage portion 113 which can be used with the aerosol generating system. The identifier may be indicative of the type of liquid 115 contained within the liquid storage portion 113.

The controller 309 determines the type of liquid storage portion 113 as the liquid storage portion identifier stored in the look-up table which is associated with the resistance value stored in the look-up table which is closest in value to the liquid storage portion resistance value determined by the controller 309, at step 709. The look-up table may be stored in a read only memory (ROM) incorporated into the processor or may be stored in a separate memory store.

Therefore the controller 309 is able to distinguish between different types of liquid storage portion 113 inserted into the aerosol generating system 100 in dependence on the determined electrical characteristic or value of the electrical component associated with the liquid storage portion 113.

Preferably, the look-up table may further comprise data representing one or more resistance values, each resistance value further associated with parameters representing a different energy profile to be applied to the heating element 119. Each resistance value is associated with a different liquid storage portion 113 identifier. This means that the aerosol generating system 100 can be configured to deliver a constant amount, for example volume or mass of aerosol to the user even when liquid storage portions 113 containing different liquids are inserted into the aerosol generating system 100.

For example, a particular liquid contained within one liquid storage portion 113 may require more energy to vaporise it than a different liquid contained within another liquid storage portion 113. By associating a resistance value or a particular liquid storage portion identifier with a heating profile stored in a look-up table, a constant amount of aerosol can be delivered to the user independent of the type of liquid stored in the liquid storage portion 113.

The controller may perform this additional optional step, shown as step 711 in Figure 9.

At step 713, the controller then applies a particular energy profile to heater 119 in dependence on the determined liquid storage portion type or identifier or independence on the determined characteristic resistance value. For example, the resistance values stored in the look-up table may be associated with an optimal voltage and current required by the heating coil to deliver to the user a particular quantity of aerosol for a particular liquid formula. In one embodiment, the liquid may comprise nicotine. The heat of vaporization of this particular liquid formula is different to the heat of vaporization required to vaporize a liquid not containing nicotine.

Therefore, the value of one or more electrical components associated with each liquid storage portion allows the controller 309 to determine the liquid storage portion identifier, and hence the type of liquid contained within the liquid storage portion 113. It also allows the controller 309 to deliver the appropriate heating profile to the heating element 119 based on the values stored in the look-up table which are associated with a particular value of electrical component.

The second portion of the electrical circuitry shown in Figure 7 is a heater circuit part, and this also shows the case when the liquid storage portion 113 is inserted into the aerosol generating system 100. The heater circuit comprises the heating element 119, one end of which is electrically connected via contacts 211 or 221, 421 to one terminal V+ of the power source. The other end of the heating element 119is electrically connected via contacts 213 or 223, 423 to a terminal of a transistor 555 or other switch having three terminals. The collector terminal of transistor 555 may be connected to the heating element, although other configurations are possible. The base terminal of the transistor 555 is electrically connected to the controller 309, while the emitter terminal of the transistor is electrically connected to the other V- terminal of the power source.

The energy supplied to the heating element 119 is controlled by the current being applied by the controller 309 to the base terminal of the transistor 555. This controls the amount of current flowing from one terminal of the battery via terminals 211 or 221, 421 through the heating coil 119 and through the collector and emitter terminals of transistor 555 via terminals 213 or 223, 423, to the other terminal of the battery.

In the electrical circuitry shown in Figure 7, one of the terminals of the battery may be a positive electrical voltage, V+, while the other terminal may be a negative electrical voltage, V-. However, this need not be the case, and it is sufficient for there to be a potential difference between the two battery terminals. Alternatively, the battery may be any current source such as a capacitor or other power supply.

Figure 10 shows an electrical circuit diagram of an aerosol generating system 100 according an alternative embodiment of the invention. In this embodiment, features in common with the embodiment shown in Figure 5, have been given like reference numerals. For the sake of clarity, the heating element 119 and transistor 555 have not been included in the circuit diagram of Figure 10. Once again, the features enclosed within the dashed line are components which are located on or within in the liquid storage portion part 113. Figure 10 also shows the case when a liquid storage portion 113 is inserted into the aerosol generating system 100.

The embodiment shown in Figure 10 has 2 electrical contacts 221, 225 or 421, 425 located on or in the liquid storage portion part 113. A resistor 804 or other electrical component is mounted on or in the liquid storage portion 113. A first terminal of resistor 804 is electrically connected to electrical contact 225, 425 on the liquid storage portion 113. A second terminal of resistor 804 is electrically connected to contact 221, 421 on the liquid storage portion 113. As shown in Figure 10, when the liquid storage portion 113 is inserted into the aerosol generating system 100, electrical contact 221 or 421 comes in to contact with electrical contact 211 mounted on the aerosol generating system. Similarly, electrical contact 225 or 425 comes in to contact with electrical contact 215 when the liquid storage portion 113 is inserted into the aerosol generating system 100.

The electrical contacts 211, 215, 221, 225, 421, 425 allow electrical signals to pass between the liquid storage portion 113 and the rest of the aerosol generating system 100, whilst also allowing the liquid storage portion 113 to be removed and replaced with a new liquid storage portion 113, once the liquid within the storage portion 113 has been depleted.

One electrical contact 211 on the aerosol generating system 100 is electrically connected by an electrically conductive wire or other conductive means to a positive terminal of a battery or power supply means, V+. This electrical contact 211 is also electrically connected to one input of the micro controller, 309.

Further, electrical contact 215 is mounted on or in the aerosol generating system 100 and is connected by an electrically conductive means such as a wire to a first terminal of a resistor, 834. The electrical contact 215 is also electrically connected to an input of a micro controller 309, such as an analogue to digital converter input of a micro controller 309, via an amplifier 806.

A second terminal of resistor 834 is electrically connected to a first terminal of a transistor 855. A second terminal of transistor 855 is electrically connected to a digital output of micro controller 309. A third terminal of transistor 855 is electrically connected to a negative terminal V- of a power supply, battery, capacitor or other means for supplying electrical energy 107. Finally, the micro controller 309 is also electrically connected to the negative terminal V-of the power supply by an electrically conductive means.

The amplifier 806 is used to amplify the signal of the voltage across resistor 834 of value R734, V834, if this voltage amplitude is sufficiently small. Therefore, the value *R804* of resistor 804 may be determined from the values of V+ and voltage across resistor 834, V834, according to the following equation, provided the value R834 of resistor 834 and the gain, A, of the amplifier are known:

$$R804 = R834(A\frac{V+}{VADC} - 1)$$

where VADC is the voltage at the ADC input of the micro controller 309. VADC is equal to the voltage V834 multiplied by the gain of the amplifier, A.

In use, the aerosol generating system according to this embodiment performs the following steps to determine the identifier or type of liquid storage portion 113. First, when the aerosol generating system power is switched on, the digital output of the micro controller 309 is set to a high level. The transistor 855, which may be a complimentary metal oxide semiconductor (CMOS) transistor is activated which makes electrical current flow through resistors 804 and 832.

In some applications, the voltage amplitude of voltage V834 is sufficiently large that an amplifier 806 is not needed. In this case, the value of resistor R804 may be determined from the values of the V+ and the voltage, V834, across resistor 834 is known according to the following equation:

$$R804 = R834(\frac{V+}{V834} - 1)$$

Then, in both cases, the voltage across resistor 834 is input into an analog to digital conversion input of the microcontroller 309. The controller determines the value *R804* of resistor 804 using one of the above equations. Then, the thus determined resistance value is then compared to a set of resistance values stored in a memory such as a read only memory. The microcontroller 309 then determines if the determined resistance value corresponds to or matches a resistance value located in a memory space of the memory. If the values match, then a stored heating element profile, which is associated with the resistance value in the memory matching the determined resistance value is used to energize the heating element 119.

In other words, the aerosol generating system 100 determines the value R804 of resistor 804. It then compares this value to a database of resistor values. Each resistor value is associated with a different heating profile. The processor then selects the heating profile associated with the resistor value in the database which corresponds to, or matches the determined value of resistor 804. The heating profile may be defined by a number of different operational parameters for the heating element.

Figure 11 shows an electrical circuit diagram of an aerosol generating system 100 according yet a further embodiment of the invention. This diagram shows the case when a liquid storage portion 113 is inserted into the aerosol generating system 100.

In this embodiment, 4 separate resistors 904, 905, 906, 907 or other electrical components are located on or in the liquid storage portion 113. A first terminal of each resistor is electrically connected to electrical contact 921. When the liquid storage portion according to this embodiment is inserted into the aerosol generating system 100, electrical contact 921 comes into electrical contact with a contact 911 mounted on or in the aerosol generating system 100. Contact 911 is electrically connected both to a positive voltage V+ of a power supply means 107 and to a micro controller 309.

A second terminal of each resistor 904, 905, 906, 907 is electrically connected to connectors 924, 925, 926, 927. Each second terminal of each resistor is electrically connected to one of the connectors 924, 925, 926, 927. Each second terminal of each resistor 904, 905, 906, 907 is electrically connected to a different connector 924, 925, 926, 927. That is to say, each second terminal of each resistor 904, 905, 906, 907 is electrically connected only to one of the connectors 924, 925, 926, 927.

Further, four electrical contacts 914, 915, 916, 917 are provided on the aerosol generating system 100 embodying the circuit diagram of Figure 11. Four resistors 934 935, 936, 937, are provided in the circuitry. A first terminal of each of the resistors 934, 935, 936, 937 on the aerosol generating system is electrically connected to connectors 914, 915, 916, 917 on the aerosol generating system. Each first terminal of each resistor 934, 935, 936, 937 is electrically connected to one of the connectors 914, 915, 916, 917. Each first terminal of each resistor 934, 935, 936, 937 is electrically connected to a different connector 914, 915, 916, 917 on the aerosol generating system 100. That is to say, each first terminal of each resistor 934, 935, 936, 937 is electrically connected to only one of the connectors 914, 915, 916, 917.

Further, each first terminal of each resistor 934, 935, 936, 937 is separately electrically connected to a controller 309.

Each second terminal of each resistor 934, 935, 936, 937 is electrically connected to one terminal of transistor or switch 955 having 3 terminals. Each second resistor terminal is connected to the same transistor terminal. A second terminal of transistor 955 is electrically connected to a digital output of controller 309. A third terminal of transistor 955 is electrically connected to a negative voltage supply V-. The controller 309 is also electrically connected to the negative voltage supply V-.

When the liquid storage portion according to this embodiment is inserted into an aerosol generation system, electrical contact is made between connector 924 on the liquid storage portion 113 and connector 914 on the aerosol generating system 100. Similarly, electrical contact is made between connector 925 on the liquid storage portion 113 and connector 915 on the aerosol generating system 100, while electrical contact is made between connector 926 on the liquid storage portion and connector 916 on the aerosol generating system, and electrical contact is made between connector 927 on the liquid storage portion 113 and connector 917 on the aerosol generating system 100.

As previously described with reference to Figure 10 above, the value of the resistors 904, 905, 906, 907 can be determined using the following equation:

$$R90X = R93X(\frac{V+}{VR93X} - 1)$$

*Where X = 4, 5, 6, 7.*

The processor 309 determines the value of each of the resistors 904, 905, 906, 907 using this equation, in a similar manner as previously described with reference to Figure 10. For example, the controller may determine that the resistors (904, 905, 906, 907) have values of (1kΩ, 2kΩ, 5kΩ, 10kΩ). This is referred to a resistor network code of (1, 2, 5, 10). With this configuration, the resistor network code is used to deliver a heating profile and the consumable type to the microcontroller. Therefore it does not require comparing the determined resistor values to stored resistor values associated with a heating profile.

The analog signals present at the analog to digital conversion ports are directly used to generate the heating profile. In this way the determined value of each of the resistors 904, 905, 906, 907 is used as a direct input to calculate the appropriate heating profile characteristics. The heating profile may be defined by a number of different operational parameters for the heating element. For example, the processor 309 uses the determined value of each resistor 904, 905, 906, 907 to directly calculate the value of each operational parameter which the processor applies to the heating element. This has the advantage that a look-up table is not required to determine what heating profile should be applied to a particular liquid storage portion since the processor 309 determines the values to be applied to the heating element based on the determined resistor values.

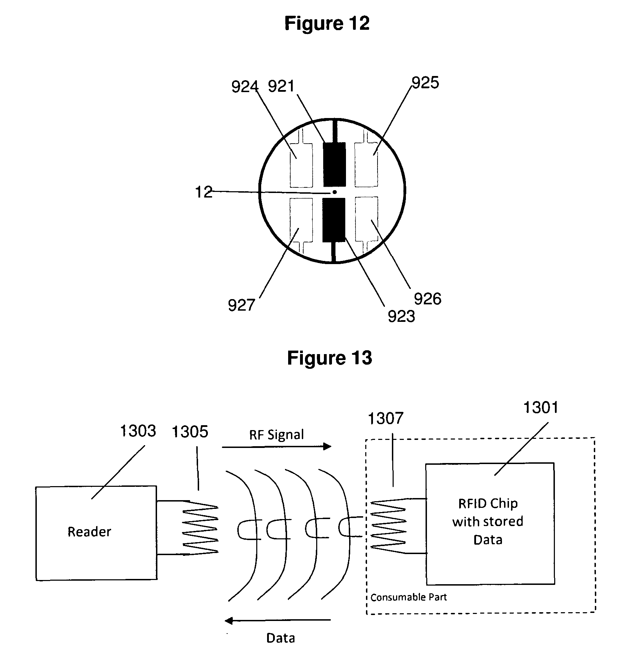

Figure 12 shows a liquid storage portion 113 according to the embodiment of the invention described with reference to Figure 11.

In this embodiment, the electrical contacts 921, 923, 924, 925, 926, 927 on the liquid storage portion 113 are arranged in a substantially rectangular or square lattice.

Figure 12 shows that the two electrical contacts 921, 923 which are closest to the central longitudinal axis of symmetry of the liquid storage portion, shown by a dot marked 12 in Figure 12, are the electrical contacts which provide electrical energy to the heating element 119. The electrical contacts 924, 925, 926, 927, which are furthest away from the longitudinal axis of symmetry of the aerosol generating system 100 are the electrical contacts which provides the electrical signals to the liquid storage portion recognition subsystem 229.

In this embodiment, the electrical contacts mounted on the aerosol generating system are arranged in a similar arrangement to the electrical contacts mounted on the liquid storage portion, shown in Figure 12, and so will not be described again in further detail.

Figure 13 is a schematic diagram of selected components of a further aerosol generating system embodying the invention.

A radio frequency identification (RFID) chip 1301 is located on or within the liquid storage portion. The chip may comprise an antenna 1307 electrically coupled to the chip. The chip 1301 and antenna 1307 may be incorporated into a single tag which may be applied by adhesive or other means to the liquid storage portion.

The chip contains a flash memory or an Electrically Erasable Programmable Read Only Memory (EEPROM) in which data such as one or more of a liquid former identification i.e. the liquid contained within the liquid storage portion 113, a cartridge serial number, and a heating profile for the liquid former.

The aerosol generating system according to this embodiment comprises an interrogator 1303 and an antenna 1305 electrically coupled to the interrogator. The interrogator 1303 and antenna 1305 may be integrated into a single processor 309 for controlling operation of the aerosol generating system 100. Alternatively the interrogator 1303 and antenna 1305 may be separate from the processor 309.

When the aerosol generating system 100 is switched on, the interrogator 1303, located on or within the aerosol generating system 100 energizes the RFID chip 1301 via antenna 1307 with a radio frequency (RF) signal emitted from the antenna 1305.

The data from the chip are then transmitted by frequency modulation emitted from the antenna 1307 located on the chip 1301 to the interrogator 1303 via the antenna 1305.

If the interrogator 1303 receives data, then the data may be stored in a memory.

The data received from the chip 1301 may comprise a liquid storage portion identifier and optionally one or more operational parameters of the heating element which the system may use to energize the heating element, or an identifier of the type of liquid contained within the liquid storage portion. The liquid storage portion identifier may be a unique identifier.

In use, the controller 309 may examine the data to determine the liquid storage portion type being used with the aerosol generating system. Therefore, the controller can distinguish between different liquid storage portions.

The controller 309 may apply a particular energy profile to heater 119 in dependence on the determined liquid storage portion type or identifier. For example, the data received by the system 100 may be optimal heating element operating parameters to deliver to the user a particular quantity of aerosol for a particular liquid formula. For example, in one embodiment, the liquid may comprise nicotine. The heat of vaporization of this particular liquid formula is different to the heat of vaporization required to vaporize a liquid not containing nicotine.

Therefore, the data encoded in a chip 1301 associated with each liquid storage portion 113 allows the controller 309 to determine the liquid storage portion identifier, and hence the type of liquid contained within the liquid storage portion 113. It also allows the controller 309 to deliver the appropriate heating profile to the heating element 119 based on the data received from the chip associated with each liquid storage portion 113.

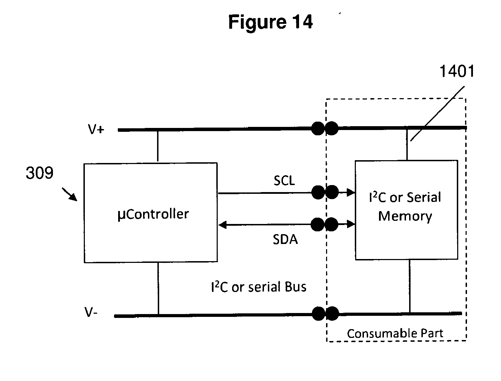

Figure 14 shows a schematic diagram of selected components of an aerosol generating system 100 embodying the invention. Data such as data identifying a liquid storage portion 113 is stored on a memory on or in the liquid storage portion.

In the embodiment shown in Figure 14, data is transferred between a controller 309 and memory 1401 using an 12C bus although other data transmission protocols are possible.

The memory 1401 is powered by electrically connecting the memory 1401 with an electrically conductive portion to one terminal of a power supply, such as a positive voltage, V+.The memory is also electrically connected with an electrically conductive portion to another terminal of a power supply, such as a negative voltage, V-.

The memory is also electrically connected to the controller 309 via a clock line, SCL, so synchronise data transfer over the data line. The memory is further electrically connected to the controller 309 via a data line, SDA, which allows data to be transferred between the memory 1401 and the controller 309.

The data stored on the memory 1401 may comprise information enabling the system 100 to distinguish between different liquid storage portions. The data may comprise operational parameters for the heating element of the smoking system which have been optimised for the particular liquid contained within the liquid storage portion 113. The data is encoded on a Programmable Read Only Memory or Flash Memory located on or in the liquid storage portion.

The data for a particular liquid storage portion, such as a heating profile, product identification or liquid storage portion identifier are stored in a flash or Erasable Programmable Read Only Memory (EPROM) memory 1401 located on or within the liquid storage portion 1301

When the aerosol generating system is switched on, the microcontroller 309 interrogates the memory 1401 via a serial communication bus using an 12C communication protocol or another serial data transfer protocol. If the data stored in cartridge present the correct format or the correct information is present, the data stored in the memory will then further be used when the aerosol generating device is used.

This embodiment has the advantage that only 2 physical connections with the device, shown as solid dark circles in figure 14, in addition to the two power connections shown in solid dark circles in Figure 14 are required for supplying electrical power to the memory 1401.

Therefore, the data encoded in the memory 1401 associated with each liquid storage portion allows the controller 309 to receive data from the memory 1401 and determine the liquid storage portion identifier, and hence the type of liquid contained within the liquid storage portion 113. It also allows the controller 309 to deliver the appropriate heating profile to the heating element 119 based on the data received from the memory 1401 associated with each liquid storage portion. The heating profile may be defined by a number of different operational parameters for the heating element.

In a further embodiment, not shown in the drawings, a 3-dimensional shape including one or more bumps, protuberances, hollows, or furrows may be formed in the liquid storage portion, or attached to the exterior or other surface of the liquid storage portion 113.

The aerosol generating system 100 includes one or more switches which are engaged by the 3-dimensional shape comprising one or more bumps on the liquid storage portion.

In this way, the particular position on the liquid storage of the bumps, protuberances, hollows, or furrows or hollows may determine whether one or more of the switches is activated.

The position of the bumps or hollows on the liquid storage portion therefore allows information about the liquid storage portion to be encoded into or onto the liquid storage portion in the form of a 3-dimensional shape. Therefore, the 3-dimentionsal shape associated with each liquid storage portion allows the controller 309 to determine the liquid storage portion identifier, and hence the type of liquid contained within the liquid storage portion 113. It also allows the controller 309 to deliver the appropriate heating profiled to the heating element based on the date decoded from the shape associated with each liquid storage portion 113.

A number of embodiments have been described and it should be understood that features described in relation to one embodiment may also apply to another embodiment, where appropriate. The scope of the present invention is defined with reference to the following claims.

**Claims**

1. A liquid storage portion (113) comprising an electrical component (504, 505, 804, 904, 905, 906, 907, 1301, 1401) for distinguishing the storage portion (113) from other liquid storage portions, the liquid storage portion (113) being configured for use in an aerosol generating system (100) having means for determining (309) an electrical characteristic of the electrical component (504, 505, 804, 904, 905, 906, 907, 1301, 1401) and means for distinguishing (309) the liquid storage portion from other liquid storage portions based on the determined electrical characteristic of the electrical component.

2. A liquid storage portion (113) according to claim 1 further comprising one or more further electrical components (505, 905, 906, 907) the components preferably being connected in series or parallel with each other.

3. A liquid storage portion (113) according to any preceding claim in which the electrical component or components comprises one or more resistors (504, 505, 804, 904, 905, 906, 907) or one or more radio frequency identification microchips (1301) or one or more microchip memories (1401).

4. A liquid storage portion (113) according to any preceding claim further comprising one or more electrical contacts (221, 223, 225, 421, 423, 425, 921, 923, 924, 925, 926, 927) for electrical connection to one or more of the electrical component or components (504, 505, 804, 904, 905, 906, 907, 1301, 1401).

5. A liquid storage portion (113) according to claim 4 in which the electrical contacts (221, 223, 225, 421, 423, 425, 921, 923, 925, 926, 927) are arranged in a substantially straight line or in a substantially triangular array or in a substantially square array or a substantially rectangular array.

6. A liquid storage portion (113) according to any preceding claim further comprising a heating element (119), the heating element (119) preferably being integral to the liquid storage portion (113).

7. An aerosol generating system (100) for receiving a liquid storage portion (113) configured for use with the aerosol generating system (100), the system comprising:

means for determining (309) an electrical characteristic of an electrical component (504, 505, 804, 904, 905, 906, 907, 1301, 1401) associated with the liquid storage portion (113); and
means for distinguishing (309) the liquid storage portion (114) from other liquid storage portions for use with the aerosol generating system (100) based on the determined electrical characteristic of the electrical component associated with the liquid storage portion (113).

8. An aerosol generating system according to claim 7 further comprising transmission means (201, 203, 205, 211, 213, 215, 221, 421, 223, 423, 224, 425, 230, 235, 1305,1307) arranged to transmit signals between the determining means (309) and the electrical component (504, 505, 804, 904, 905, 906, 907, 1301, 1401).

9. An aerosol generating system (100) according to claim 8 in which the determining means (309) further comprises one or more further electrical components (834, 934, 935, 936, 937).

10. An aerosol generating system (100) according to any one of claims 7 to 9 further comprising a memory for storing a look-up table, the look-up table comprising data representing one or more electrical characteristics of one or more electrical components, each electrical characteristic associated with data identifying a liquid storage portion (113).

11. An aerosol generating system (100) according to claim 10 further comprising a means (309) for searching the look-up table.

12. A liquid storage portion (113) comprising a 3-dimensional object for distinguishing the storage portion (113) from other liquid storage portions, the liquid storage portion (113) being configured for use in an aerosol generating system (100) having means for determining (309) a characteristic of the 3-dimensional object and means for distinguishing (309) the liquid storage portion from other liquid storage portions based on the determined characteristic of the 3-dimensional object.

13. An aerosol generating system (100) for receiving a liquid storage portion configured for use with the aerosol generating system (100), the system comprising:

means for determining (309) a characteristic of a 3-dimensional object associated with the liquid storage portion (113); and
means for distinguishing (309) the liquid storage portion (113) from other liquid storage portions for use with the aerosol generating system (100) based on the determined characteristic of the 3-dimensinoal object associated with the liquid storage portion (113).

14. A method for distinguishing between types of liquid storage portion used with an aerosol generating system (100), the method comprising the steps of:

determining (309) an electrical characteristic of an electrical component (504, 505, 804, 904, 905, 906, 907, 1301, 1401) associated with the liquid storage portion (113);
distinguishing (309) the liquid storage portion (114) from other liquid storage portions for use with the aerosol generating system (100) based on the determined electrical characteristic of the electrical component associated with the liquid storage portion (113).

15. A method for distinguishing between types of liquid storage portion used with an aerosol generating system (100), the method comprising the steps of:

determining (309) a characteristic of a 3-dimensional object associated with the liquid storage portion (113);
distinguishing (309) the liquid storage portion (114) from other liquid storage portions for use with the aerosol generating system (100) based on the determined characteristic of the e3-dimensional object associated with the liquid storage portion (113).

**Figure 1**

## Figure 2

## Figure 3

# Figure 4

# Figure 5

# Figure 6

**Figure 7**

## Figure 8

Switch On ~ 601

Apply voltage to contacts 211, 215 ~ 603

Measure current flow ~ 605

Current > predetermined value? ~ 607

No

Yes

Allow heater to be energised ~ 609

# Figure 9

Switch On — 601

↓

Apply voltage to contacts 211, 215 — 603

↓

Measure current flow — 605

↓

Determine resistance from voltage and current — 707

↓

Determine cartridge identifier from resistance — 709

↓

Look up energy profile for determined cartridge identifier — 711

↓

Apply energy profile to heater based on determined cartridge identifier — 713

## Figure 10

## Figure 11

## Figure 12

## Figure 13

# Figure 14

EP 2 399 636 A1

Europäisches Patentamt
European Patent Office
Office européen des brevets

# EUROPEAN SEARCH REPORT

Application Number

EP 10 25 1135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 772 166 A1 (CANON KK [JP]) 11 April 2007 (2007-04-11) | 1-4,7-9, 14 | INV. A61M11/04 A61M15/06 A24F47/00 |
| Y | * paragraph [0040] - paragraph [0041] * ----- | 5,6,10, 11 | |
| Y | US 2009/095287 A1 (EMARLOU HAMID [US]) 16 April 2009 (2009-04-16) * paragraph [0029] - paragraph [0031] * ----- | 6 | |
| Y | WO 2005/025654 A1 (INJET DIGITAL AEROSOLS LTD [AU]; REINHOLD OLAF [US]; LACKEY ROBERT P []) 24 March 2005 (2005-03-24) * page 43, line 12 - page 43, line 18 * ----- | 10,11 | |
| Y | US 2006/118612 A1 (CHRISTOFFERSEN LASSE W [DK] ET AL CHRISTOFFERSEN LASSE WENGEL [DK] ET) 8 June 2006 (2006-06-08) * paragraph [0025]; figures * * paragraph [0030] * * paragraph [0116] * * paragraph [0117] * ----- | 5 | |
| A,D | WO 2007/078273 A1 (AUGITE INC [US]; LIU ZHEN [CN]) 12 July 2007 (2007-07-12) * abstract; figures * ----- | 1-11,14 | TECHNICAL FIELDS SEARCHED (IPC) A61M A24F |
| X | WO 03/059424 A1 (AEROGEN INC [US]) 24 July 2003 (2003-07-24) * figures 2,5-6 * * page 14, line 1 - page 14, line 15 * ----- | 12,13,15 | |
| A | WO 2006/084543 A1 (PARI GMBH [DE]; BORGSCHULTE MARKUS [DE]; ACHTZEHNER WOLFGANG [DE]) 17 August 2006 (2006-08-17) * figure 2 * * paragraph [0031] * ----- | 12,13,15 | |

The present search report has been drawn up for all claims

| Place of search | Date of completion of the search | Examiner |
|---|---|---|
| Munich | 17 May 2011 | Valfort, Cyril |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

26

Europäisches
Patentamt

European
Patent Office

Office européen
des brevets

Application Number

EP 10 25 1135

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-11, 14

   A liquid storage portion comprising an electrical component, an aerosol generating system for receiving a liquid storage portion and the coresponding method.

   ---

2. claims: 12, 13, 15

   A liquid storage portion comprising a 3-dimensional object for distinguishing the storage portion, an aerosol generating system and corresponding method.

   ---

EP 2 399 636 A1

## ANNEX TO THE EUROPEAN SEARCH REPORT
## ON EUROPEAN PATENT APPLICATION NO.

EP 10 25 1135

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2011

| Patent document cited in search report | | Publication date | Patent family member(s) | | Publication date |
|---|---|---|---|---|---|
| EP 1772166 | A1 | 11-04-2007 | CN | 1943810 A | 11-04-2007 |
| | | | JP | 2007097787 A | 19-04-2007 |
| | | | US | 2007076067 A1 | 05-04-2007 |
| US 2009095287 | A1 | 16-04-2009 | NONE | | |
| WO 2005025654 | A1 | 24-03-2005 | AU | 2003260166 A1 | 06-04-2005 |
| | | | EP | 1670531 A1 | 21-06-2006 |
| | | | JP | 2007505644 T | 15-03-2007 |
| US 2006118612 | A1 | 08-06-2006 | AT | 402688 T | 15-08-2008 |
| | | | EP | 1608305 A1 | 28-12-2005 |
| WO 2007078273 | A1 | 12-07-2007 | NONE | | |
| WO 03059424 | A1 | 24-07-2003 | AU | 2003203043 A1 | 30-07-2003 |
| | | | EP | 1474196 A1 | 10-11-2004 |
| | | | JP | 2005514991 T | 26-05-2005 |
| | | | JP | 2006150093 A | 15-06-2006 |
| | | | US | 2003140921 A1 | 31-07-2003 |
| | | | US | 2008060641 A1 | 13-03-2008 |
| | | | US | 2005172954 A1 | 11-08-2005 |
| WO 2006084543 | A1 | 17-08-2006 | DE | 102005006372 A1 | 24-08-2006 |
| | | | EP | 1850897 A1 | 07-11-2007 |
| | | | US | 2009120431 A1 | 14-05-2009 |

EPO FORM P0459

For more details about this annex : see Official Journal of the European Patent Office, No. 12/82

**EP 2 399 636 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007078273 A